# EUROPEAN PATENT SPECIFICATION

(11) **EP 1 130 085 B1**
(45) Date of publication and mention of the grant of the patent: **05.10.2005**
(21) Application number: 01500028.4
(22) Date of filing: 01.02.2001
(51) Int. Cl.: C12N 1/16, C12N 1/22, C12N 9/42, C12P 7/10

(54) **Procedure for the production of ethanol from lignocellulosic biomass using a heat-tolerant yeast**
Verfahren zur Herstellung von Ethanol aus Lignocellulose-haltiger Biomasse unter Verwendung von thermostabiler Hefe
Procédé de production d'éthanol à partir d'une biomasse lignocellulosique au moyen d'une levure thermotolérante

(30) Priority: 24.02.2000 ES 200000439
(43) Date of publication of application: 05.09.2001
(73) Proprietor: CENTRO DE INVESTIGACIONES ENERGETICAS MEDIOAMBIENTALES Y TECNOLOGICAS (C.I.E.M.A.T.), E-28040 Madrid (ES)
(72) Inventor: Ballesteros Perdices, Ignacio, 28026 Madrid (ES); Ballesteros Perdices, Mercedes, 28035 Madrid (ES); Oliva Dominguez, José Miguel, 28942 Fuenlabrada, Madrid (ES); Carrasco Garcia, Juan Esteban, 28863 Cobena, Madrid (ES)
(74) Representative: Del Santo Abril, Natividad

(56) References cited:
- WO-A-91/10740
- BALLESTEROS I ET AL: "Effect of surfactants and zeolites on simultaneous saccharification and fermentation of steam-exploded poplar biomass to ethanol." APPLIED BIOCHEMISTRY AND BIOTECHNOLOGY, vol. 70-72, 1 April 1998 (1998-04-01), pages 369-381, XP001010109 ISSN: 0273-2289
- BALLESTEROS I ET AL: "OPTIMIZATION OF THE SIMULTANEOUS SACCHARIFICATION AND FERMENTATION PROCESS USING THERMOTOLERANT YEASTS" APPLIED BIOCHEMISTRY AND BIOTECHNOLOGY,US,CLIFTON, NJ, vol. 39, no. 40, 1993, pages 201-211, XP000197347 ISSN: 0273-2289
- BOLLOK MONIKA ET AL: "Simultaneous saccharification and fermentation of steam-pretreated spruce to ethanol." APPLIED BIOCHEMISTRY AND BIOTECHNOLOGY, vol. 84-86, 1 April 2000 (2000-04-01), pages 69-80, XP001010142 ISSN: 0273-2289
- Jeffries Tom: "Biotechnology for biomass conversion" (1997-03-27), http://calvin.biotech.wisc.edu/jeffries/bioproc- essing/bioconversion.html
- BALLESTEROS M. ET AL: 'Ethanol production from paper material using a simultaneous saccharification and fermentation system in a fed-batch basis' WORLD JOURNAL OF MICROBIOLOGY & BIOTECHNOLOGY vol. 18, no. 6, August 2002, pages 559 - 561

## Description

This invention refers to an improved procedure for obtaining ethanol from lignocellulosic biomass by means of a saccharification and simultaneous fermentation process. More specifically, it refers to a procedure in which lignocellulosic biomass is subject, in an initial phase, to a hydrothermal steam explosion pre-treatment, followed by simultaneous hydrolysis (using commercial cellulases) and fermentation with a new heat-tolerant strain of *Kluyveromyces marxianus* yeast.

### Background of the invention

Obtaining ethanol fuel from biomass contributes to safety in the supply of energy, since it is an alternative to fuel of a fossil origin. It also contributes to regional development, with the resulting benefits associated to the creation of employment. As is the case for other renewable energies, the production and use of bioethanol in the transport industry has environmental advantages over fuel derived from oil, since the emission of contaminants is reduced and the greenhouse effect is not increased.

The production of ethanol from renewable raw materials can use a large variety of substrates. The raw materials used to produce this type of alcohol are hydrocarbonated products with sugar or starch, liable to undergo a fermentation process, either directly (saccharose) or after hydrolysis (starch, inulin). Crops such as beetroot and sugar cane (the first group) and cereals such as corn (the second group) are some examples of the raw materials that are currently being used for the production of bioethanol. Lignocellulosic materials (organic and forest residues and herbaceous and ligneous crops) can also be used as raw material for the production of bioethanol.

Both for sugary and starchy substrates, the total cost of the process is dependent on the price of the raw material, which is between 60 and 70% of the total cost of the product. To improve the competitiveness of bioethanol fuel compared with fuels based on oil, new processes need to be developed in order to obtain the product from cheaper substrates such as certain lignocellulosic raw materials.

Before being transformed into ethanol, the cellulosic fraction of lignocellulosic materials requires hydrolysis in order to be changed into fermentable glucose by micro-organisms. This stage, which can be performed by means of acid or enzymatic catalysts, is a problem, because of the chemical stability of the cellulose chain and the protection of plant tissue afforded by lignin, which makes the process costly in economic and energy terms.

Enzymatic hydrolysis of cellulose has at least three potential advantages over acid-catalyst processes:
- Greater yields
- Lower equipment costs, since it is carried out at atmospheric pressure and low temperatures.
- No toxic substances are produced as a result of the degradation of sugars, which could be an obstacle for fermentation.
However, and because of the structure of the lignocellulosic materials, carbohydrates are not directly accessible to hydrolytic enzymes and a series of prior treatments are therefore required to improve the yield of the hydrolysis. The heavy inhibition experienced by the cellulases from the accumulation of the final products of the reaction, basically cellobiose and glucose, is another factor that limits the yield of the hydrolysis.

In general, the processes for obtaining ethanol from lignocellulosic biomass include the following stages: pre-treatment, hydrolysis of the cellulose and fermentation of the glucose. The purpose of the pre-treatment stage is to facilitate the penetration and spread of the enzymes and micro-organisms.

### Pre-treatment

Since 1919, when Beckmann patented an alkaline pre-treatment based on impregnation with sodium hydroxide, which improved the digestibility of straw, many pre-treatments have been developed for lignocellulosic materials.

Of the pre-treatments tested, hydrothermal processes appear to be among the most effective for improving the accessibility of these materials. An example of these hydrothermal processes is described in Shell International Research's Spanish patent ES87/6829, which uses steam at a temperature of 200-250°C in a hermetically sealed reactor to treat previously ground biomass. In this process, the reactor is cooled gradually to ambient temperature once the biomass is treated. However, and although this improves the accessibility of the biomass to an eventual enzymatic attack, the version of the hydrothermal treatment that includes a sudden depressurisation of the reactor, called steam explosion treatment, has been shown to be one of the most effective when it comes to facilitating the eventual action of cellulolytic enzymes. Steam explosion is a thermal-mechanical-chemical process that combines the presence of heat (as steam), mechanical forces (shearing effect) and chemical action (hydrolysis). The result is the alteration of the microfibrillar packing inside the cell wall and the rupture of the fibre, which causes an increase in the accessibility of the cellulose to hydrolytic enzymes. The optimum temperature and reaction time conditions vary depending on the kind of material.

Discontinuous steam explosion treatment was patented in 1929 by Mason (United States patent US 1.655.618) for the production of boards of timber, and it combines a thermal treatment with steam and the mechanical disorganisation of lignocellulosic fibre. In this process, the wooden splinters are treated with steam at a pressure of 3,5 MPa or higher, in a vertical steel cylinder. Once the treatment is completed, the material is violently discharged from the base of the cylinder. This process combines the effects on the lignocellulosic material of high pressures and temperatures together with the final and sudden decompression. The effect that this treatment has is a combination of physical (segregation and rupture of the lignocellulosic fibres) and chemical (de-polymerisation and rupture of the C-O-C links) modifications. During steam treatment, most of the hemicellulose is hydrolysed to oligomers soluble in water or free sugars.

There are very different applications for steam explosion treatment. For example, United States patent US 4.136.207 (1979) describes the use of this kind of pre-treatment to increase the digestibility of hard woods such as poplar and birch by ruminants. In this case, STAKE technology is used, operating continuously in a high-pressure tubular reactor, at temperatures between 200 and 250°C and for different treatment times.

In the discontinuous steam explosion process developed by IOTECH Corporation, known as "flash hydrolysis", the wood is ground to a small particle size and subject to temperatures and pressures close to 230°C and 500 psi, and once these conditions are reached, it is suddenly discharged from the reactor. The wood's organic acids control the pH and acetic acid is always present in the gaseous effluent. The design of the reactor in what is popularly known as the IOTECH process is described in United States patent US 4.461.648.

Regarding another application of steam explosion treatment for lignocellulosic materials, Canadian patent CA 1.212.505 describes the application of a combination of the STAKE and IOTECH steam explosion processes to obtain paper paste from hard wood with high yields.

In this invention, steam explosion treatment has been used to increase the digestibility of the cellulose to enzymatic hydrolysis by means of microbial cellulases in a simultaneous saccharification and fermentation process (SSF). This use of steam explosion treatment as the pre-treatment in an SSF process is a new application of this treatment and is one of the novelties of this invention.

The basic objective of this pre-treatment is to reduce the crystallinity of the cellulose and to dissociate the hemicellulose-cellulose complex. The digestibility of the cellulose increases with the degree of severity of the pre-treatment, and this increase in digestibility is directly related to the increase in the available surface area (ASA) of the cellulose fibre, which facilitates the eventual enzymatic attack by cellulases. This increase of the ASA is a result of the partial or total elimination of the hemicellulose and the lignin.

Research carried out on the increase of the accessibility of the substrate, after steam explosion treatment, has been focussed on the study of a series of factors related to the substrate, such as the distribution of the pore size, the degree of crystallinity, the degree of polymerisation or the residual xylan content, which determine its final effectiveness (K.K.Y. Wong et al., Biotechnol. Bioeng. 31, 447 (1988); H. L. Chum et al., Biotechnol. Bioeng. 31, 643, (1988)). The first researches focussed their work on the effect of sudden de-pressurisation on the rupture of the cellulose in experiments at high temperatures (220-270°C) and short treatment times (40-90 seconds). More recent work (Wright, J.D. SERI/TP-231-3310, 1988; Schwald et al., in: Steam explosion Techniques. Fundamentals and Industrial Applications, Facher, Marzetti and Crecenzy (eds.), pages 308-320 (1989)), has shown that the use of lower temperatures (no higher than 200-220°C) and longer treatment times (between 5 and 10 minutes) produce appropriate solubilisation rates and also avoid the possibility of a certain amount of pyrolysis being produced, which could give rise to inhibitory products. The conditions applied in this application are along these lines, and it has been determined that they lead to a greater recovery of glucose in the residue (Ballesteros et al., in: Biomass for Energy, Environment, Agriculture and Industry, Chartier, Beenackers and Grassi (eds.), Vol. 3., pages 1953-1958 (1995)). Enzymatic hydrolysis of cellulose and fermentation of glucose. Simultaneous saccharification and fermentation process (SSF) .

Enzymatic hydrolysis of cellulose is carried out by means of a mixture of enzymatic activities that are known as a group as cellulolytic enzymes or cellulases. One of the enzymes, called endoglucanase, is adsorbed on the surface of the cellulose and attacks the inside of the polymer chain, breaking it at one point. A second enzyme, called exoglucanase, then frees two units of glucose, called cellobiose, from the nonreducing end of the chain. The cellobiose produced in this reaction can accumulate in the medium and significantly inhibit the exoglucanase activity. The third enzymatic activity, the β-glucosidase, splits these two sugar units to free the glucose that is later fermented to ethanol. Once again, the glucose can accumulate in the medium and inhibit the effect of the -glucosidase, then producing an accumulation of cellobiose, which as we have mentioned before, inhibits the exoglucanase activity.

Although there are different types of micro-organisms that can produce cellulases, including bacteria and different kinds of fungi, what are generally used are genetically altered strains of the filamentous fungus *Trichoderma ressei,* since they have greater yields. Traditional cellulase production methods are discontinuous, using insoluble sources of carbon, both as inducers and as substrates, for the growth of the fungus and enzyme production. In these systems, the speed of growth and the rate of cellulase production are limited, because the fungus has to secrete the cellulases and carry out a slow enzymatic hydrolysis of the solid to obtain the necessary carbon. The best results have generally been obtained in operations with discontinuous feeding, in which the solid substrate, for example Solka Floc or pre-treated biomass, is slowly added to the fermentation deposit so that it does not contain too much substrate (Watson et al., Biotech. Lett., 6, 667, 1984). According to Wright, J.D. (SERI/TP-231-3310, 1988), average productivity using Solka Floc and pre-treated agricultural residues is around 50 IU/l.h. The improvement of these productivity rates, and the increase of the specific activity of these enzymes, which is by nature extremely low, are tow of the primary objectives of present research on the subject.

In the conventional method for producing ethanol from lignocellulosic materials, a cellulase is added to the material pre-treated in a reactor for the saccharification of the cellulose to glucose, and once this reaction is completed, the glucose is fermented to ethanol in a second reactor. This process, called separate saccharification and fermentation, implies two different stages in the process of obtaining ethanol. Using this method, the conversion rate of cellulose to glucose is low, because of the inhibition that the accumulation of glucose and cellobiose causes to the action of the enzyme complex, and consequently, large amounts of non-hydrolysed cellulosic residues are obtained which have a low ethanol yield. In fact, according to Wright, J.D. (SERI/TP-231-3310, 1988), this inhibition of the final product is the most significant disadvantage of the separate saccharification and fermentation process, and is one of the main factors responsible for its high cost, since large amounts of cellulolytic enzyme are used in an attempt to solve this problem.

British patent GB 2 186 289 B described a procedure with several stages of separate saccharification and fermentation to obtain ethanol from leguminous grasses. The stages are: homogenising the vegetable material, hydrolysing this material with an inorganic base, making the pre-treated material react with β-cellulase, filtering the reaction media, fermenting the filtrate with a microbial system to produce ethanol and separating the ethanol produced.

One of the most interesting options for the previous method is the simultaneous saccharification and fermentation (SSF) method. In this process, the presence of the yeasts together with the cellulolytic enzyme reduces the accumulation of sugars in the reactor and it is therefore possible to obtain greater yields and saccharification rates than with the separate hydrolysis and fermentation process. Another additional advantage is the use of a single fermentation deposit for the entire process, thus reducing the cost of the investment involved. The presence of ethanol in the medium also makes the mixture less liable to be invaded by undesired micro-organisms (Wyman, C.E. Bioresource Technology, 50, 3-16, 1994).

In the simultaneous hydrolysis and fermentation process the fermentation and saccharification must be compatible and have a similar pH, temperature and optimum substrate temperature. One problem associated to the SSF process is the different optimum temperature for saccharification and fermentation. Since the optimum temperature for saccharification is within a 45-50°C range, the use of heat-tolerant yeasts is recommendable for simultaneous SSF processes.

Over recent years, research has been performed and a bibliography written on the different strains of yeast that are capable of growing at temperatures above 40°C, although there is not much literature on ethanol fermentations with high yields using these micro-organisms. Szczodrak and Targonski (Biotechnology and Bioengineering, vol. 31, pages 300-303, 1988), tested a total of 58 strains of yeasts from 12 families for their capacity to grow and ferment sugars at temperatures of 40-46°C. Several strains from the *Saccharomyces, Kluyveromyces* and *Fabospora* families were selected for their capacity to ferment glucose, galactose and mannose at 40, 43 and 46°C, respectively. The greatest ethanol yields were found in two strains of the *F*. *Fragilis* and *K*. *Fragilis* species, which produced 56 and 35 g/l of ethanol from 140 g/l of glucose, at 43 and 46°C, respectively.

In this invention, we use a new strain of the *Kluyveromyces marxianus* species, CECT 10875, obtained by means of chemical mutagenesis and subsequent selection, which is capable of fermenting the glucose produced by the hydrolysis of the cellulose to ethanol at 42°C, and the yields of which have been improved compared to those of the original strain.

Research carried out in recent years on the SSF process has lead to significant improvements in ethanol production which have been the subject of several patents. These studies have primarily been based on the selection of the micro-organism, the optimum concentration of the enzyme and different substrate pre-treatments, but mainly considering discontinuous processes. For example, MAXOL & C.B.'s patent WO 96/37627 describes a discontinuous SSF process for ethanol production from a vegetable material, in which a mixture of hemicellulases and commercial cellulases is used for the saccharification process, and a yeast from the *Candida, Kluyveromyces*, *Pichia* and *Saccharomyces* families, or a mixture of them, is used to ferment the different sugars produced. In this process, the vegetable material is subjected to pre-treatment with an acid or a base, although this has the disadvantage that the material has previously to be ground to a size of approximately 1 mm, which represents a high energy cost. The materials of vegetable origin used are very heterogeneous, and when a material is used that is similar to that of this invention, for example, forage straw, the yields obtained are around ten times less than those obtained with the procedure described in this invention, which uses a heat-tolerant yeast. This low yield could be attributed to the fact that the process uses a temperature of 35°C, which, although it is adequate for fermentation with the selected yeasts, is outside the optimum range for the saccharification process.

Other examples of SSF processes to obtain ethanol described in the bibliography are from Wyman et al., (Biotech Bioeng. Symp., pages 21-238, 1998) and Spindler et al., (Appl. Biochem. Biotechnol., 28/29, pages 773, 1991), which use a medium that is a mixture of *Bretanomyces clousenii* and *Saccharomyces cerevisiae,* which ferments both the cellobiose and the glucose produced by the hydrolysis of the cellulose. This process, which takes place at 37°C, has a treatment time of 7 days, which can be considered very high for this kind of process.

A previous work of the authors (Applied Biochemistry and Biotechnology vol. 70-72, pp369-381) using the strain *Kluyveromyces marxianus* EMS-26 describes residence times of 144 hours in an ethanol production process starting out from pre-treated woody biomaases. The SSF process described in this invention uses the new heat-tolerant strain of *Kluyveromyces marxianus* CECT-10875 enabling a considerable reduction in the fermentation time taking place at the same temperature of 42°C, which represents a major improvement with regard to the aforementioned work. The ethanol yield of this new strain (grams of ethanol/ grams of glucose contained in the raw material) makes it possible to achieve concentrations of ethanol similar to those attained with K marxianus EMS-26, but in just 72 hours. Therefore the volumetric productivity (quantity of ethanol per unit of volume and time) of the process is far greater than that obtained with the EMS-26 strain. Another significant improvement of the proposed process is the use of particle sizes of up to 30 mm in the pre-treatment phase. Despite the fact that the Biotechnology document for biomass conversion (1997-03-27) (http://calvin.biotech.wisc.edu/jeffries/bioprocessing/bioco nversion.html) mentions that the particle sizes employed for the biomass conversion processes range from 3/4" to 1/B", this is not so evident when a steam explosion treatment is used as a pre-treatment prior to the SSF process. There is documentary evidence to show that the particle size of the biomass has an important effect on possible modifications that may have taken place during pre-treatment, which at the same time effect the enzyme's accessibility to the substrate during the SSF. As a result, the possibility of using large particle sizes of up to 30 mm in pre-treatment without affecting the final hydrolysis and fermentation yields represents an innovation and a major improvement, since this brings about a significant reduction in the energy required for grinding, thereby reducing costs.

As for continuous SSF systems, conventional designs are continuously shaken tank reactors, arranged in a series or in cascade formation. One of the greatest disadvantages of this type of system is its high cost, since it requires long treatment times and vigorous shaking, which leads to the de-naturalisation of the enzymes and the need to replace them every so often. The Nguyen, Q.A. patent WO 98/30710 describes a system that is a tower bioreactor, based on flow-piston reactor technology, which leads to a significant reduction of the volume of the fermentation deposits and the energy required for shaking. This system allows for the use of mixes with a high content in suspended solids, such as the pre-treated lignocellulosic materials, because in the previously described systems they are only applicable to aqueous mixes. Nevertheless, the bibliography does not yet contain a description of continuous SSF process development that obtain high yields and production rates.

### Description of the invention

The procedure covered by this invention is a discontinuous procedure to obtain ethanol from lignocellulosic biomass, which includes a steam explosion pre-treatment and the simultaneous saccharification (by means of commercial cellulases) and fermentation (using a new heat-tolerant yeast, particularly *Kluyveromyces marxianus* CECT 10875) of cellulose to ethanol. The process is carried out at 42°C. Shaking at 150 rpm and treatment time is 72 h. After the pre-treatment, 1,000 g of biomass with a cellulose content of 30-40% (not susceptible to an enzyme attack) gives 270-360 g of cellulose susceptible of being hydrolysed. This cellulose is transformed by means of a SSF process in 90-120 g of ethanol.

### Brief description of the drawings

To complete the previous description, and with a view to providing a better understanding of the characteristics of the invention, there will be a detailed description of a preferred embodiment, based on a set of orientative but not restrictive drawings that are attached to this description and represent the following:

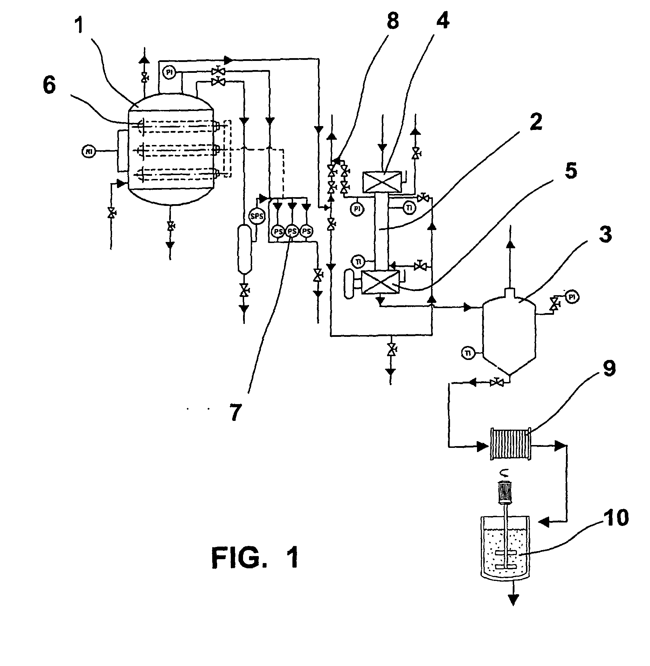

Figure 1 shows a diagram of the procedure that constitutes the invention.

### Description of a preferred embodiment

The raw material used in this invention is a material that contains mostly cellulose, such as forestry and agricultural residues, paper paste, lignocellulosic crop biomass and the organic fraction of domestic waste. Normally, this material has been dried by air and contains between 10-15% humidity.

Although the material has to be ground before the pre-treatment, the particle sizes required (15-30 mm) are considerably larger than those used in other reactor designs, reducing the energy costs associated to the grinding. This heat treatment with steam leads to condensation and the creation of a humid lignocellulosic mass. Self-hydrolysis occurs because the temperature is high enough to thermodynamically force the dissociation of the liquid water, creating an acid medium that overcomes the energy barriers of the hydrolysis. The introduction of steam into the structures of the lignocellulosic materials is guaranteed, because the diffusion of the steam phase is greater than the diffusion of the liquid phase. First the steam penetrates and then it is condensed. This capillary water is in equilibrium because of the high pressure. When the material is de-pressurised the capillary water rapidly evaporates, which has the mechanical effect of segregating and breaking some fibres, probably with a greater impact on the weakest regions (amorphous cellulose). The mechanical effect is clearly caused by the rapid evaporation of the internal water. This evaporation creates shearing forces that produce the separation of the fibres.

The installation used for the pre-treatment in this invention is made up of three units: a steam accumulator (1), a steam explosion reactor (2) and a discharge cyclone (3), the characteristics of which are described as follows. See figure 1.

The steam accumulator (1) has to supply steam at a temperature of 245°C and a pressure between a and 3 MPa to the steam explosion reactor (2). It consists of a pressure recipient equipped with several electric resistances (6). At the steam outlet there is a vent leading to the atmosphere, closed by two valves (8), so that there is an air escape during the initial filling, when it is being set at pressure and operating temperature. The pressure switches (7) that act on the resistances (6) are set at scaled pressures and each one acts on one resistance (6), switching it off or on depending on whether the set value is reached or not.

The steam explosion reactor (2) is the chamber where the lignocellulosic biomass is compressed and suddenly de-pressurised. It consists of a 3" diameter stainless steel 316 vertical pipe, limited by two 3" diameter stainless steel 316 throttle valves. The input valve (4) on the top of the chamber opens and closes by hand and is used to load the ground lignocellulosic biomass in the reactor (2). The output valve (5), on the bottom of the chamber, opens by a triggering and spring device in less than 1 second. The mixture of steam and biomass is thus discharged violently, and passes through a pipe that carries it to the cyclone (3). The reactor chamber (2), valves and discharge pipe are insulated with 70 mm thick mineral wool, in order to reduce as much as possible the condensation of the steam during the compression-expansion process.
The discharge cyclone (3) is built in stainless steel 316 and has a cylindrical part with a diameter of 16" and a conical part which, coming down from the cylindrical part and at an angle of 60°, ends in a DN-80 and PN-16 flange neck, on which there is a valve of the hot set type through which the material expanded in the reactor is removed. The upper edge of the cylindrical part of the cyclone ends in a 16" flange equipped with locking tabs and fasteners that hold down the eyebolts that fasten the blind flange that acts as the cyclone lid. The cyclone has a thermometer and a manometer.

For the SSF a fermentation deposit (10) is used, built in stainless steel and equipped with mechanical shaking, pH and temperature control. There is a filter press (9) at the inlet.

The process is as follows:

The ground material is introduced in the steam explosion equipment (2) and subject to a pressure of between 1 and 3 MPa and temperatures between 190 and 230°C, by means of the injection of saturated steam from the steam accumulator (1) and for a period of time of between 1 and 10 minutes, depending on the raw material used. Once the pre-treatment stage is over, the mixture of steam and lignocellulosic biomass that is expelled enters the discharge cyclone (3), horizontally and tangentially, where the volatile elements are eliminated and it is filtered to separate the liquid fraction from the solid fraction. The liquid fraction basically contains the majority of the hemicellulosic sugars (xylose, arabinose, mannose and galactose), the products of the degradation of these sugars (furfural, hydroxymethylfurfural), organic acids (mainly acetic) and phenolic compounds produced by the solubilisation of the lignin. The solid fraction basically contains cellulose and lignin and this is used as the raw material for the hydrolysis of the cellulose to glucose. This glucose is the substrate for fermentation to ethanol.

After leaving the filter press (9), the material is introduced in the fermentation deposit (10) in a solid/liquid ratio that varies depending on the material that makes up the lignocellulosic biomass, between 8-15% (w/v). Once the material has been introduced in the fermentation deposit and been diluted adequately, a commercial cellulolytic compound is added (such as CELLUCLAST 1.5L, from the firm NOVO-NORDISK) in a concentration of 15 Filter Paper Units (FPU) per gram of cellulose and 12.6 International Units per gram of β-glucosidase enzyme cellulose, such as NOVOZYME 188 from NOVO-NORDISK, both re-suspended in citrate buffer pH 4.8. The enzymatic activities are determined following the methods described by the IUPAC (International Union of Pure and Applied Chemistry), described by Ghose, T.K. (Pure and Appl. Chem., Vol. 59, number 2, pages 257-268, 1987).

Because of the previously mentioned final product inhibition of the cellulolytic complexes, a SSF process such as the one described in this invention, in which the glucose is eliminated from the medium as it is produced, represents a significant improvement in the yield of the hydrolysis. For this purpose, this invention uses a new heat-tolerant strain of *Kluyveromyces marxianus* (CECT 10875), which provides a fundamental advantage, since it makes the action of the enzymatic complex compatible with fermentation at close to optimum temperatures in both cases. This new strain has been obtained by chemical mutagenesis from the DER-26 *Kluyveromyces marxianus* strain belonging to the collection of the CIEMAT's Department of Renewable Energies. This original strain was subject to different doses of the alkylating agent ethylmethanesulphonate, and then selected for its capacity to grow and ferment glucose to ethanol at temperatures in the 42-45°C range, as described in Applied Biochemistry and Biotechnology, Vol. 39/40, pages 201-211 (1993). This strain is deposited in the Colección Española de Cultivos Tipo (CECT - Spanish Medium Collection) with order number 10875.

In the SSF process that is part of this invention, the fermentation deposit (10) that contains the pre-treated biomass and the cellulolytic complex, as described previously, is inoculated with a suspension of a medium of the *Kluyveromyces marxianus* CECT 10875 grown at 42°C for 16 h in a concentration of 10% (v/v). This mix is shaken at 150 r.p.m. for 72 h at 42° C. After this time, it has been shown that there is no increase in the concentration of ethanol, so after 72 hours the process is considered to be complete and the final concentration of ethanol and the residual sugars in the medium are determined by HPLC.

## Claims

1. Procedure for the production of ethanol from lignocellulosic biomass **characterised in that** it includes the stages of:
Grinding the lignocellulosic biomass to a particle size of 15-30 mm.
Subjecting the ground lignocellulosic biomass to steam explosion pre-treatment, maintaining at a pressure of between 1 and 3 MPa and a temperature between 190 and 230°C, for a period of time of between 1 and 10 minutes, depending on the type of material used and later provoking rapid de-pressurisation.
Collecting the pre-treated material and separating the liquid and solid fractions by filtration, and introducing the solid fraction in the fermentation deposit (10).
Adding a cellulase to the fermentation deposit (10) in a concentration of 15 FPU per gram of cellulose and 12.6 International Units of β-glucosidase enzyme.
Inoculating the fermentation deposit (10) with a suspension of a culture of the heat-tolerant yeast *Kluyveromyces marxianus* CECT 10875.
Shaking the mixture for 72 h at 42°C.
Determining the concentration of ethanol and residual sugars in the mix, once the reaction is complete.

2. Procedure for the production of ethanol from lignocellulosic biomass, in accordance with claim 1, **characterised in that** the humidity content of the lignocellulosic biomass is between 10 and 15%.

3. Procedure for the production of ethanol from lignocellulosic biomass, in accordance with claim 1, **characterised in that** the solid fraction that is introduced into the fermentation deposit has a solid/liquid ratio that varies between 8 and 15% (w/v).

4. Procedure for the production of ethanol from lignocellulosic biomass, in accordance with claim 1, **characterised in that** the cellulase and the β-glucosidase are dissolved in citrate buffer pH 4.8.

5. Procedure for the production of ethanol from lignocellulosic biomass, in accordance with claim 1, **characterised in that** the *Kluyveromyces marxianus* CECT 10875 inoculant is at a concentration of 10% v/v.

6. Procedure for the production of ethanol from lignocellulosic biomass, in accordance with claim 1, **characterised in that** the mixture is shaken at 150 r.p.m.

## Patentansprüche

1. Verfahren zur Herstellung von Ethanol aus lignocellulosehaltiger Biomasse **dadurch gekennzeichnet, dass** es die folgenden Stufen umfasst:
Mahlen der lignocellulosehaltigen Biomasse zu einer Partikelgröße von 15-30 mm.
Unterziehen der gemahlenen lignocellulosehaltigen Biomasse einer Dampfexplosionvorbehandlung, wobei sie unter einem Druck zwischen 1 und 3 MPa und auf eine Temperatur zwischen 190 und 230°C während eines Zeitraumes zwischen 1 und 10 Minuten gehalten wird, je nach eingesetztem Materialtyp und späteres Auslösen eines schnellen Druckablassen.
Einsammeln des vorbehandelten Materials und Trennen der flüssigen und festen Fraktionen durch Filtration und
Einbringen der festen Fraktion in die Fermentationsablagerung (10).
Zusetzen einer Zellulase zu der Fermentationsablagerung (10) in einer Konzentration von 15 FPU per Gramm von Zellulose und 12,6 internationale Einheiten von β-Glukosidase-Enzym.
Inokulieren der Fermentationsablagerung (10) mit einer Suspension einer Kultur von der thermotolerante Hefe *Kluyveromyces marxianus* CECT 10875.
Rühren der Mischung 72 Stunden bei 42°C.
Bestimmen der Konzentration von Ethanol und restlichen Zuckern in der Mischung einmal die Reaktion zu Ende ist.

2. Verfahren zur Herstellung von Ethanol aus lignocellulosehaltiger Biomasse nach Anspruch 1 **dadurch gekennzeichnet, dass** der Feuchtigkeitsgehalt der lignocellulosehaltigen Biomasse zwischen 10 und 15% liegt.

3. Verfahren zur Herstellung von Ethanol aus lignocellulosehaltiger Biomasse nach Anspruch 1 **dadurch gekennzeichnet, dass** die feste Fraktion, die in die Fermentationsablagerung eingebracht ist, ein Flüssig/Fest-Verhältnis hat, das zwischen 8 und 15% (w/v) variiert.

4. Verfahren zur Herstellung von Ethanol aus lignocellulosehaltiger Biomasse nach Anspruch 1 **dadurch gekennzeichnet, dass** die Zellulase und die β-Glukosidase in Zitratpuffer pH 4,8 gelöst werden.

5. Verfahren zur Herstellung von Ethanol aus lignocellulosehaltiger Biomasse nach Anspruch 1 **dadurch gekennzeichnet, dass** das *Kluyveromyces marxianus* CECT 10875 Inokulum in einer Konzentration von 10% v/v liegt.

6. Verfahren zur Herstellung von Ethanol aus lignocellulosehaltiger Biomasse nach Anspruch 1 **dadurch gekennzeichnet, dass** die Mischung bei 150 Upm gerührt wird.

## Revendications

1. Procédé pour la production d'éthanol à partir de biomasse lignocellulosique **caractérisé en ce qu'**il inclut les étapes de:
Moudre la biomasse lignocellulosique jusqu'à une taille de particule de 15-30 mm,
Soumettre la biomasse lignocellulosique moulue à un prétraitement avec explosion de vapeur, maintenant une pression entre 1 et 3 MPa et une température entre 190 et 230°C, pendant une période de temps entre 1 et 10 minutes, en fonction du type de matériau utilisé et postérieurement déclencher une dépressurisation rapide,
Ramasser le matériau prétraité et séparer les fractions liquides et solides par filtration et introduire la fraction solide dans le dépôt de fermentation (10),
Ajouter une cellulase au dépôt de fermentation (10) à une concentration de 15 UFP par gramme de cellulose et 12,6 unités internationales d'enzyme bêta-glucosidase,
Inoculer le dépôt de fermentation (10) avec une suspension d'une culture de la levure thermotolérante *Kluyveromyces marxianus* CECT 10875,
Agiter le mélange pendant 72 h à 42°C,
Déterminer la concentration d'éthanol et de sucres résiduels dans le mélange, une fois la réaction est complétée.

2. Procédé pour la production d'éthanol à partir de biomasse lignocellulosique selon la revendication 1, **caractérisé en ce que** le contenu en humidité de la biomasse lignocellulosique se trouve entre 10 et 15%.

3. Procédé pour la production d'éthanol à partir de biomasse lignocellulosique selon la revendication 1, **caractérisé en ce que** la fraction solide qui est introduite dans le dépôt de fermentation a un rapport solide/liquide qui varie entre 8 et 15% (w/v).

4. Procédé pour la production d'éthanol à partir de biomasse lignocellulosique selon la revendication 1, **caractérisé en ce que** la cellulase et la bêta-glucosidase se dissolvent dans du tampon citrate, pH 4,8.

5. Procédé pour la production d'éthanol à partir de biomasse lignocellulosique selon la revendication 1, **caractérisé en ce que** l'inoculant *Kluyveromyces marxianus* CECT 10875 se trouve à une concentration de 10% v/v.

6. Procédé pour la production d'éthanol à partir de biomasse lignocellulosique selon la revendication 1, **caractérisé en ce que** le mélange est agité à 150 rpm.
